# EUROPEAN PATENT APPLICATION

(11) **EP 1 443 438 A1**
(43) Date of publication of application: **04.08.2004**
(21) Application number: 02768060.2
(22) Date of filing: 25.09.2002
(51) Int. Cl.: G06F 17/60

(54) **DNA PROVIDING SYSTEM**

(30) Priority: 25.09.2001 JP 2001290889
(71) Applicant: Toyo Kohan Co., Ltd., Tokyo 102-8447 (JP); Takahashi, Kojiro, Hiroshima-shi, Hiroshima 734-0015 (JP)
(72) Inventor: TANGA, Michifumi, c/o TOYO KOHAN CO., LTD., Kudamatsu-shi, Yamaguchi 744-8611 (JP); TAKAHASHI, Kojiro, Hiroshima-shi, Hiroshima 734-0015 (JP)
(74) Representative: VOSSIUS & PARTNER
(86) International application number: PCT/JP2002/009870
(87) International publication number: WO 2003/027927

(57) **Abstract**

It is intended to enable the provision of efficient DNA data and DNA chip s without being restricted to time, place, environment and other factors. Namel y, a DNA providing system consisting of an apparatus for selecting and purchas ing DNA data and chips which has a provided data receiving unit for receiving DNA data and/or DNA chip data provided; a provided data accumulating/selecti ng unit for accumulating the provided data and selecting data desired by a user from among the provided data; a selected data inputting unit for inputting the DNA data and/or the DNA chip selection data selected by the user; a selected data outputting/accumulating unit for enabling the output of the selected data an d accumulating the same; a selected data transmitting unit for outputting the sel ected data outward; and an indicating/controlling unit for indicating and controlli ng the provided data, the selected data or the like; and an apparatus for providi ng DNA date/chips which has a selected data receiving unit for receiving the se lected data; a storing/managing unit for storing the selected data, DNA data, DN A samples for DNA chips and the DNA chips; a provided data outputting/accu mulating unit for enabling output of the provided data corresponding to the sele cted data outward and accumulating the same; a provided data transmitting unit for outputting the provided data outward; a DNA chip constructing unit for cons tructing DNA chips desired by a user in case of lacking the same; and an indic ating/controlling unit for indicating and controlling the provided data, the selecte d data or the like.

## Description

### Technical Field

The present invention relates to a DNA providing system "Gene Net Bank (GNB in short) " for quickly providing DNA data (base sequence data and the like inclusive of gene polymorphism and the like such as SNP (Single Nucleotide Polymorphism), VNTR (Variable Number of Tandem Repeat) polymorphism, a microsatellite polymorphism, and an insertion/deletion-type polymorphism which users desire) in accordance with techniques of a gene analysis, a gene diagnosis/cure, a gene utilization, a gene storage and the like and for providing DNA chips in which DNA samples such as genes containing the aforementioned DNA data, DNA (Deoxyribonucleic Acid) fragments or the like are immobilized on chips.

### Background Art

DNA is a main body of a gene, has a functional portion which relates not only to determination of constituents of a living body but also to diseases, and is being found to be useful in screening or the like of a gene diagnosis and medicaments. Under the condition in which a large-scale DNA decoding of an entire living body such as analysis of human genome is also progressing, a speed of function analysis of DNA has increased, and application of DNA data for the gene analysis, the gene diagnosis/cure, a new drug development or the like is becoming popular.

Some international DNA data banks for allowing such DNA data to be common (impartial) have been established and the DNA data have been put in commercial use. However, a DNA providing system "Gene Net Bank (GNP) in which the DNA data and DNA samples thereof comprehensively correspond to each other does not exist at present.

In order to relate function data of DNA, for example, related with diseases with actual base sequence data of the DNA (inclusive of gene polymorphism data such as SNP, VNTR polymorphism, microsatellite polymorphism, an insertion/deletion-type polymorphism and the like), researchers in universities and laboratories and people engaged in research and development in companies have been solving the functions of DNA and base sequence data thereof one after another at the sacrifice of tremendous cost and time.

Development and application of DNA chips as measures for reducing the tremendous cost and time in research and development have rapidly been advancing. Due to convenience of the gene analysis, the gene diagnosis and the like to be performed by using DNA immobilized on a chip (glass or silicon substrate, particularly glass substrate), a practical application of the DNA chip in the field of the gene utilization·storage related with biochemistry, cell biology, molecular biology, molecular genetics, gene medical science, an actual gene medical field, a food problem and an environment problem has been progressing.

Further, for the purpose of allowing not only such analysis-type DNA chip, but also a minute amount of precious DNA sample to be obtained from cancerous tissue cell to be in a re-analyzable form whenever needed, a storage-type DNA chip which can semi-permanently store the gene sample or DNA segment thereof utilizing a diamond substrate has been developing.

A user who desires to obtain a DNA chip in which a specific DNA is immobilized, after solving the base sequence related with a given disease, can establish and prepare DNA having the sequence and, then, immobilize the thus-established DNA by being put on a chip by the user itself or being contracted out to prepare a DNA chip.

However, there is a problem in that, when the DNA chips are prepared by the user itself, an expensive apparatus is necessary and, even when a relatively small number of one or two types of DNA is necessary, cost, time and labor are required. On the other hand, when the preparation of the DNA chips is contracted out and there is unsolved base sequence of the DNA, a given time period for solving the unsolved base sequence of DNA is necessary and, accordingly, it is difficult to quickly obtain the DNA chips.

Further, it is a present situation that a large burden is put on the user who desires to use a portion of DNA fragments on the available DNA chip on which many DNA fragments are immobilized and efficiency is unfavorable.

The invention has been achieved to solve the aforementioned problems and an object thereof relates to a DNA providing system "Gene Net Bank (GNP)" which not only quickly provides DNA (data: for example, base sequence data inclusive of gene polymorphism data and the like such as SNP, VNTR polymorphism, microsatellite polymorphism, and an insertion/deletion-type polymorphism) but also provides a DNA chip in which gene containing such DNA data and a DNA fragment sample thereof are immobilized on a chip.

### Disclosure of the Invention

In order to solve the aforementioned problems, an intensive study has been conducted and, as a result, it has been found that the problems are solved by establishing a system comprising an apparatus for selecting and purchasing a specified DNA data/chips and an apparatus for providing DNA data/chips to achieve the present invention.

Namely, according to the invention as described in Claim 1, there is provided a DNA providing system (or Gene Net Bank (GNB in short)), comprising:
an apparatus for selecting and purchasing DNA data/chips comprising a provided data receiving unit for receiving DNA data and/or DNA chip data (hereinafter referred to as "provided data") provided via a communication line;
a provided data accumulating/selecting unit for accumulating the provided data received by the provided data receiving unit and selecting the provided data desired by a user from among the thus-accumulated provided data;
a selected data inputting unit for inputting the DNA data and/or DNA chip selection data (hereinafter referred to as "selected data") selected by the user from among the provided data of the provided data accumulating/selecting unit;
a selected data outputting/accumulating unit for enabling an output of the selected data selected by the selected data inputting unit, and accumulating the same thus enabled;
a selected data transmitting unit for outputting the selected data from the selected data outputting/accumulating unit outward; and
an indicating unit/controlling unit for indicating and controlling the provided data, the selected data or the like, and
an apparatus for providing DNA data/chips comprising a selected data receiving unit for receiving the selected data from the selected data transmitting unit of the apparatus for selecting and purchasing DNA data/chips;
a storing/managing unit for storing the selected data, the DNA data, DNA samples for the DNA chips, and the DNA chips;
a provided data outputting/accumulating unit for enabling an output of the provided data corresponding to the selected data outward from the storing/managing unit, and accumulating the same thus enabled;
a provided data transmitting unit for outputting the provided data from the provided data outputting/accumulating unit outward;
a DNA chip constructing unit for constructing DNA chips desired by the user when the DNA chips are not stored in a DNA chip storing unit of the storing/managing unit; and
an indicating unit/controlling unit for indicating and controlling the provided data, the selected data or the like.

As described in Claim 2, it is preferable that, in the apparatus for selecting and purchasing DNA data/chips, the selected data inputting unit further has a function of inputting new DNA data and/or DNA chips which do not exist in the provided data (hereinafter referred to as "new DNA data and/or DNA chips");
the selected data outputting/accumulating unit has a function of enabling an output of the new DNA data and/or DNA chips and accumulating the same thus enabled; and
the selected data transmitting unit has a function of outputting the new DNA data and/or DNA chips outward.

Further, as described in Claim 3, it is preferable that, in the apparatus for providing DNA data/chips, the selected data receiving unit has a function of receiving the new DNA data and/or DNA chips from the selected data transmitting unit of the apparatus for selecting and purchasing DNA data/chips;
when the new DNA data and/or DNA chips which are stored as provided data are in a state of being not transmitted to the apparatus for selecting and purchasing DNA data/chips, the storing/managing unit further has a function of giving instructions for transmitting the same; and
the provided data outputting/accumulating unit further has a function of enabling an output of the new DNA data and/or DNA chips outward from the storing/managing unit as the provided data and accumulating the same thus enabled.

Still further, as described in Claim 4, it is preferable that, when DNA samples for constructing the DNA chips desired by the user are not stored, the apparatus for providing DNA data/chips further comprises a DNA data and/or DNA sample purchasing unit for purchasing DNA data and/or DNA samples from other providing institutions or the user.

Furthermore, as described in Claim 5, it is preferable that the apparatus for providing DNA data/chips further comprises a DNA chip data analysis unit for performing a data analysis of DNA chips on which purchased DNA samples are immobilized.

Still furthermore, as described in Claim 6, it is preferable that transmission/reception of the selected data between the selected data transmitting unit of the apparatus for selecting and purchasing DNA data/chips and the selected data receiving unit of the apparatus for providing DNA data/chips are conducted in a non-contact manner.

Further, as described in Claim 7, it is preferable that transmission/reception of the provided data between the provided data transmitting unit of the apparatus for providing DNA data/chips and the provided data receiving unit of the apparatus for selecting and purchasing DNA data/chips are conducted in a non-contact manner.

Still further, as described in Claim 8, it is preferable that the storing/managing unit of the apparatus for providing DNA data/chips comprises a selected data storing unit, a DNA data storing unit, a DNA sample storing unit, and a DNA chip storing unit.

Furthermore, as described in Claim 9, it is preferable that the DNA chip constructing unit of the apparatus for providing DNA data/chips comprises means for DNA replication, a chip activating unit and a DNA immobilizing unit.

### Brief Description of the Drawings

FIG. 1 is a block diagram showing an outline of a system according to the present invention as described in Claim 1;
FIG. 2 is a block diagram showing an outline of a DNA providing system according to the invention as described in Claim 1;
FIG. 3 is an explanatory diagram showing a state of use of a system according to a system of the invention as described in Claim 1;
FIG. 4 is an explanatory diagram showing a state of use of a system according to the invention as described in Claim 1;
FIG. 5 is an explanatory diagram showing a state of use of a system according to the invention as described in Claim 1;
FIG. 6 is an explanatory diagram showing an example of a provided data search screen;
FIG. 7 is an explanatory diagram showing an example of a provided data view; and
FIG. 8 is an explanatory diagram showing an example of a selection screen.

### Best Mode for Carrying Out the Invention

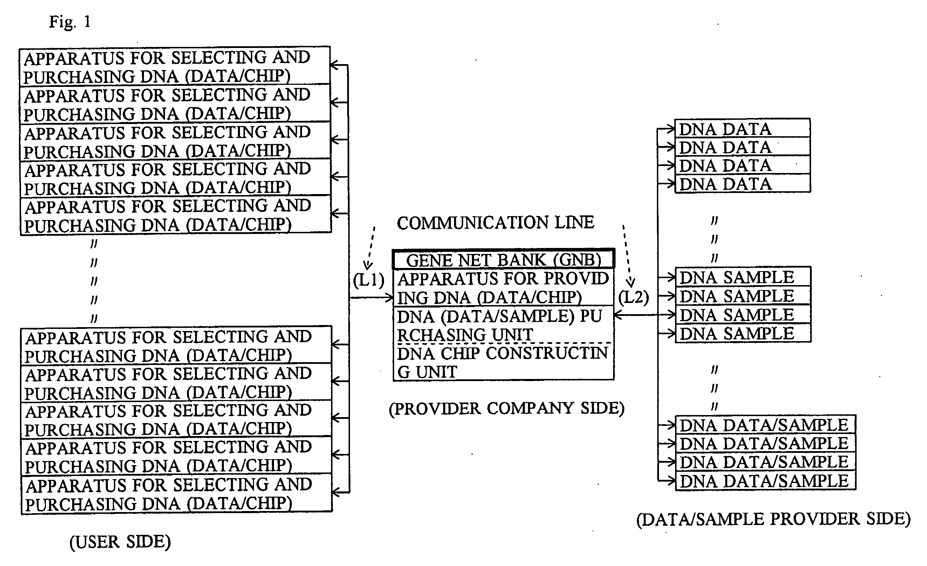

A DNA providing system "Gene Net Bank (GNB in short)" of the present invention according to Claim 1 will be described in detail below with reference to FIGS. 1 to 3.

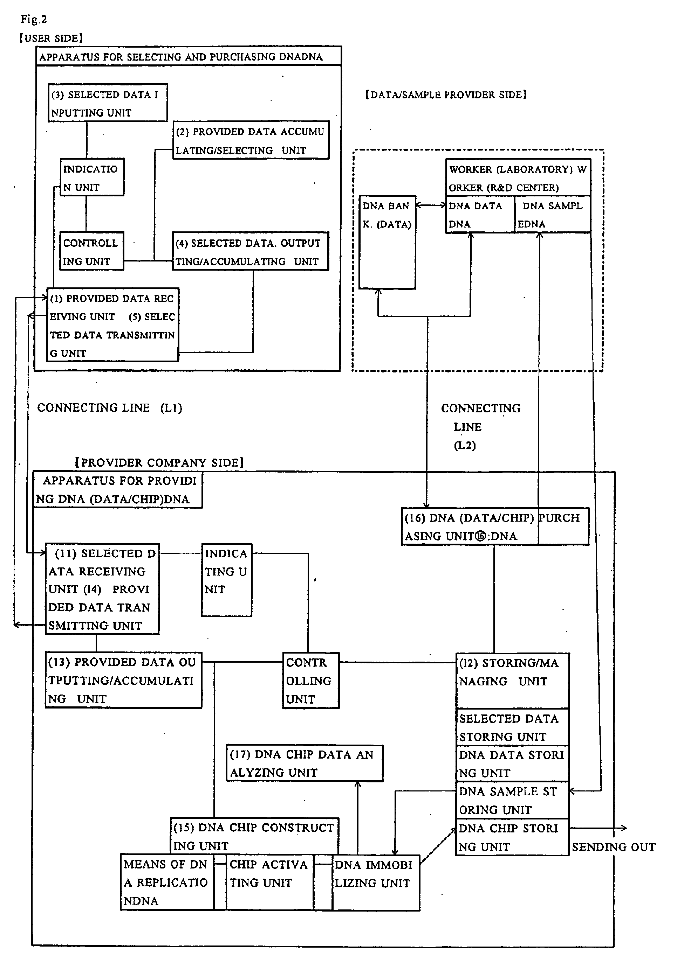

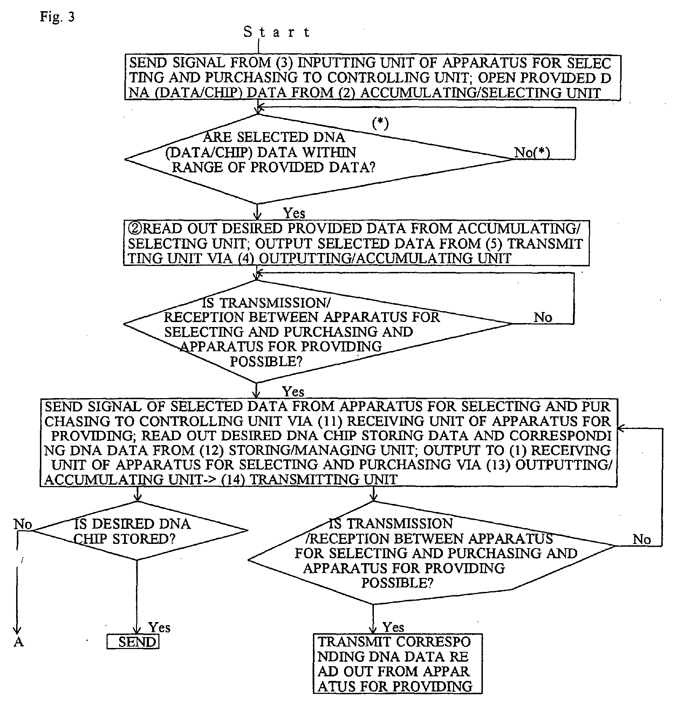

FIG. 1 shows an outline of a DNA providing system "Gene Net Bank (GNB)" according to Claim 1. FIG. 2 is a block diagram showing an outline of a DNA providing system according to the "Gene Net Bank (GNB)". FIGS. 3 to 5 are each an explanatory diagram of a state of use of the "Gene Net Bank (GNB)" shown by a flowchart.

### (1) outline of Gene Net Bank (GNB)

Firstly, an outline of a DNA providing system "Gene Net Bank (GNB)" is described with reference to FIG. 1.

As described in FIG. 1, the DNA providing system "Gene Net Bank (GNB)" comprises a main system via a communication circuit (L1) and an auxiliary system via a communication circuit (L2).

The term "main system" as used herein means a system in which, after a contract for sale and purchase of DNA data and/or DNA chips via the communication circuit (L1) is made between a provider company side of DNA data/DNA chips having an apparatus for providing DNA data/chips comprising a DNA data and/or DNA sample purchasing unit and a DNA chip constructing unit and a user side having an apparatus for selecting and purchasing DNA data/chips, a shipment of the DNA data and/or DNA chips and payment for a charge (DNA data providing cost, and DNA chip providing cost in accordance with a raw material thereof) are executed.

On the other hand, the term "auxiliary system" as used herein means that, when DNA data desired by a user side or a material for a DNA chip is not in stock in a provider company side, after a contract for sale and purchase of DNA data and/or DNA sample via a communication line (L2) is made between a provider company and a provider side of the DNA data and/or DNA sample, a shipment of the DNA data and/or DNA sample and payment of a charge are performed.

The apparatus for selecting and purchasing DNA data/chips is a communication apparatus or the like such as a personal computer and is placed within a reach of the user such as a laboratory, and a researcher oneself.

On the other hand, the apparatus for providing DNA data/chips is an apparatus for reading data related with the DNA data and/or DNA chips and, then, providing the thus-read data to the user and is owned by a provider company which is a supplier of the DNA data/chips.

Further, the DNA data and/or DNA sample provider side is any of other providing institutions or the user and provides a DNA sample which will be a material for the DNA data and/or DNA chips. As for the providers, for example, a commercial DNA bank (data) is mentioned. As for the providers who provide not only the DNA data but also the DNA samples, researchers (research centers) and people engaged in development (developing centers), including users, are mentioned.

### (2) Apparatus for selecting and purchasing DNA data/chips

An apparatus for selecting and purchasing DNA data/chips will be described below.

The apparatus for selecting and purchasing DNA data/chips comprises, as shown in FIG. 2, a controlling unit, (1): a provided data receiving unit, (2): a provided data accumulating/selecting unit, (3): a selected data inputting unit, (4): a selected data outputting/accumulating unit, (5): a selected data transmitting unit, and a indicating unit for indicating the provided data, selected data or the like.

The controlling unit comprises a personal computer or a microprocessor and controls the apparatus for selecting and purchasing DNA data/chips as a whole.

(2): the provided data accumulating/selecting unit not only has a function of accumulating provided data which the provided data receiving unit receives but also has a function of selecting provided data desired by a user from among the thus-accumulated provided data.

(3): the selected data inputting part is a unit for inputting DNA data and/or DNA chip selection data (hereinafter referred to as "selected data") selected by a user from among the provided data of (2): the provided data accumulating/selecting unit.

Further, (4): the selected data outputting/accumulating unit has a function of enabling an output of the selected data selected by the selected data inputting unit and accumulating the thus-enabled selected data.

(2): the provided data accumulating/selecting unit and (4): the selected data outputting/accumulating unit are constituted by a large-capacity memory medium such as a hard disk capable of performing anytime-writing/readout or a magneto-optical disk.

(1): the provided data receiving unit connected with the communication circuit (L1) receives data related with DNA data and/or DNA chips (hereinafter referred to as "provided data") transmitted via the communication circuit (L1) from a provider company which is a provider of the DNA data/chips, namely, DNA data or the like corresponding to a portion related with a disease or various types of living body constituents from among DNA (data: base sequence data inclusive of gene polymorphism or the like such as SNP, VNTR polymorphism, microsatellite polymorphism, an insertion/deletion-type polymorphism or the like) or a DNA sample thereof which can be provided from the provider company.

(5): the selected data transmitting unit connected with the communication circuit (L1) also transmits DNA data and/or DNA chip selection data (hereinafter referred to as "selected data") selected by the user from among the provided data to the provider company via the communication circuit (L1).

Further, the apparatus for selecting and purchasing DNA data/chips is arranged such that, even when the user desires new DNA data and/or DNA chips which do not exist in the provided data (hereinafter referred to as "new DNA data and/or DNA chips"), the apparatus can correspond to such desire. Namely, the apparatus is arranged such that it can accept request data or the like for relevant new DNA data and/or DNA chips.

On this occasion, when the user inputs the new DNA data and/or DNA chips in (3): the selected data inputting unit, (4): the selected data outputting/accumulating unit enables an output of such data and, then, accumulates the thus-enabled data and, thereafter, (5): the selected data transmitting unit outputs such data outward. Namely, the user transmits the new DNA data and/or DNA chips to (11): the selected data receiving unit of the apparatus for providing DNA data/chips and can sound as to whether provided data related with DNA data and/or DNA chips can be constructed or whether, in a case of DNA chips, it is possible to correspond to a special order for constructing a mother chip.

Still further, against such sounding, (1): the provided data receiving unit can receive an answer or a response to a request for the new DNA data and/or DNA chips from the apparatus for providing DNA data/chips. (1): the provided data receiving unit also receives auxiliary data concerning billing control data or the like of cost or the like required for a provision of the new DNA data and/or DNA chips or the special order.

The controlling unit of the apparatus for selecting and purchasing DNA data/chips controls writing/accumulation of provided data or the like received by (1): the provided data receiving unit into (2): the provided data accumulating/selecting unit or controls output/accumulation of the selected data or the like inputted into (3): the selected data inputting unit in (4): the selected data outputting/accumulating unit.

As for the communication circu it (L1), a public line such as a telephone line, a satellite communication line, or the like can appropriately be utilized in accordance with a condition of installation of the apparatus for selecting and purchasing DNA data/chips.

The provided data are transmitted from the apparatus for providing DNA data/chips owned by the provider company and are preferably data-compressed. Such data-compression can save not only a communication cost by reducing time for use of the communication line but also capacity of (2): the provided data accumulating/selecting unit.

(2): the provided data accumulating/selecting unit of the apparatus for selecting and purchasing DNA data/chips accumulates the provided data received by the provided data receiving unit and selects provided data desired by the user from among the thus-accumulated provided data.

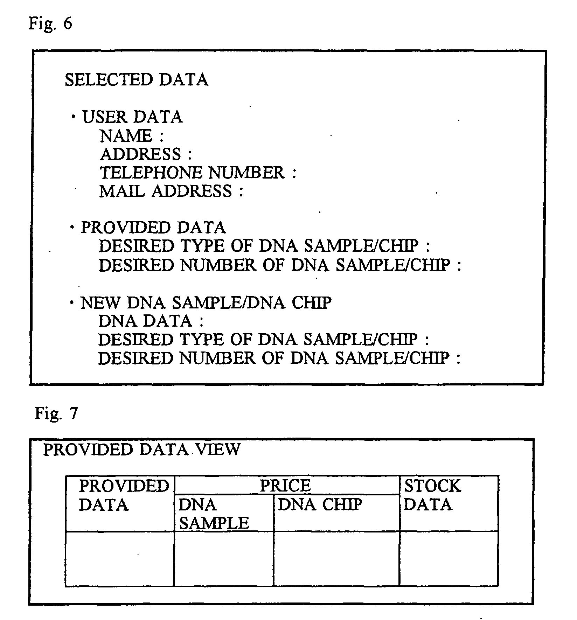

For example, as shown in FIG. 6, a search screen of the provided data is shown on an appropriate display of the indicating unit and, then, a name of living thing, a name of enzyme, a name of disease and the like can appropriately be selected. When the user inputs data related with desired DNA data, a provided data view is shown in which data of type, cost, storage status and the like of DNA are indicated. These provided data are transmitted from the apparatus for providing DNA data/chips of the provider company via the communication circuit (L1).

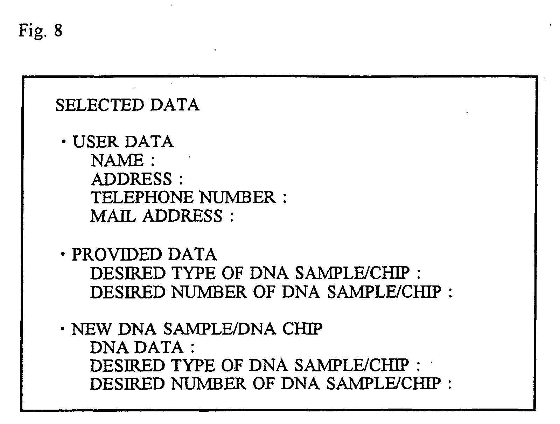

Then, when the user clicks any one of the provided data, the screen is changed to a subsequent selection screen as shown in FIG. 8 which is arranged such that it is possible to select and indicate which is required, DNA data such as a type and number of DNA or DNA chips (inclusive of a case in which the user desires both of them) desired by the user and selected from among DNA data and/or DNA chip provided data. Further, selection part into which a contact such as a name, an address, a mail address or the like of the user can be inputted is provided. Further, when the user desires to purchase new DNA data and/or DNA chips which do not exist in the provided data view, a selection part for writing such data or the like is provided.

These selection parts are connected to a signal inputting unit of the controlling unit and the user can proceed to subsequent procedures.

In (5): the selected data transmitting unit of the apparatus for selecting and purchasing DNA data/chips, when the user inputs selected data at (3) selected data inputting unit, (4): the selected data outputting/accumulating unit enables an output of the selected data and, then, accumulates the thus-enabled selected data and, thereafter, the selected data are outputted outward. Namely, the selected data can be transmitted to (11): the selected data receiving unit of the apparatus for providing DNA data/chips.

Further, the selected data of the DNA data and/or DNA chips which (5): the selected data transmitting units transmits are also preferably to be compressed. Such compressed data can save not only a communication cost by reducing the time of use of the communication line but also capacity.

Still further, the indicating unit of the apparatus for selecting and purchasing DNA data/chips is a part which indicates selected data or the like select-inputted from (3): the selected data inputting unit from among DNA data and/or DNA chip provided data which (2): the provided data accumulating/selecting unit receives via (1): provided data receiving unit or the provided data of (2): the provided data accumulating/selecting unit. As required, the indicating unit is connected to a printer or the like, thereby enabling such data to be printed.

### (3) Apparatus for providing DNA data/chips

An apparatus for providing DNA data/chips will be described below.

The apparatus for providing DNA data/chips comprises, as shown in FIG. 2, a controlling unit, (11): a selected data receiving unit, (12) a storing/managing unit comprising a selected data storing unit, a DNA data storing unit, a DNA sample storing unit and a DNA chip storing unit, (13): a provided data outputting/accumulating unit, (14): a provided data transmitting unit, (15): a DNA chip constructing unit comprising means for DNA replication, a chip activating unit and a DNA immobilizing unit, (16): a DNA data and/or DNA sample purchasing unit, (17): a DNA chip data analysis unit, and an indicating unit for indicating selected data, provided data, storing/managing data or the like.

The controlling unit comprises a personal computer or a microprocessor and controls the apparatus for providing DNA data/chips as a whole.

Further, (12): the storing/managing unit and (13): the provided data outputting/accumulating unit are constructed by a large-capacity memory medium such as a RAM, a flash memory or the like which are capable of performing anytime-writing/readout.

(11): the selected data receiving unit of the apparatus for providing DNA data/chips receives selected data which (4): the selected data outputting unit of the apparatus for selecting and purchasing DNA data/chips outputs, (5): the selected data transmitting unit transmits and the user selected in the apparatus for providing DNA data/chips. Further, it receives request data of new DNA data and/or DNA chips. The selected data or the like which are received by (11): the selected data receiving unit are accumulated in the selected data storing unit of (12): the storing/managing unit by the controlling unit.

Further, a communication line (L1) between (5): the selected data transmitting unit of the apparatus for selecting and purchasing DNA data/chips and (11): the selected data receiving unit of the apparatus for providing DNA data/chips may transmit/receive data by being connected mechanically and electrically and, on this occasion, it is preferable to utilize an infrared line communication, an optical communication, an electromagnetic induction communication or the like. By such utilization, a DNA providing system high in security and reliability can be constructed.

(12): the storing/managing unit of the apparatus for providing DNA data/chips comprises a selected data storing unit for storing DNA data and/or DNA chip selection data from the apparatus for selecting and purchasing DNA data/chips, a DNA data storing unit for DNA data which can be provided, a DNA sample storing unit for storing DNA sample itself for constructing such DNA chips and a state of managing the DNA sample, and a DNA chip storing unit for storing the thus-constructed DNA chips themselves and a state of managing the DNA chips and, also, performs work for shipping the DNA chips to the user.

Further, in a case in which the user requests new DNA data and/or DNA chip, when the new DNA data and/or DNA chips which are stored as provided data are in a state of being not transmitted to the apparatus for selecting and purchasing DNA data/chips, (12): the storing/managing unit has a function of giving instructions for transmitting the same.

Still further, when DNA data or DNA sample does not exist, (12): the storing/managing unit has a function of conveying the fact to the DNA data and/or DNA sample purchasing unit. Also, it has a function of sending a sample purchased by (16): the DNA data and/or DNA sample purchasing unit and data of such purchasing to the DNA chip constructing unit and, then, instructing construction of the DNA chips.

DNA data/DNA chips to be targeted for being provided are not specifically limited, but mentioned are DNA data and DNA chips of, for example, libraries of oligonucleotides, RNAs (mRNA, tRNA, rRNA or the like), cDNA (complementary DNA) and gDNA (chromosome DNA), and gene/DNA fragment or the like prepared by performing a PCR amplification, plasmid amplification or the like on the library.

(13): the provided data outputting/accumulating unit of the apparatus for providing DNA data/chips not only enables an output of the provided data corresponding to the selected data outward from the storing/managing unit but also accumulates the thus-enabled provided data. (14): the provided data transmitting unit outputs this provided data outward by utilizing the communication line (L1).

Further, data of the selected data to be outputted by (13): the provided data outputting/accumulating unit are also preferably compressed. Such data-compression can save not only a communication cost by reducing time for use of the communication line but also capacity.

The indicating unit of the apparatus for providing DNA data/chips is a part which indicates selected data which (12): the storing/managing unit receives via (11): selected data receiving unit or the provided data or the like outputted from (13): the provided data outputting/accumulating unit. As required, the indicating unit is connected to a printer or the like, thereby enabling such data to be printed.

When a DNA chip desired by the user is not stored in the DNA chip storing unit of the storing/managing unit, (15): the DNA chip constructing unit of the apparatus for providing DNA data/chips has a function of constructing the DNA chip and comprises means for DNA replication, a chip activating unit and a DNA immobilizing unit. (15): this DNA chip constructing unit is ordinarily owned by a provider company which is a provider of DNA data/chips, but may be owned by other institutions and the provider company may take a form of contracting out.

Further, when a DNA sample for constructing the DNA chip desired by the user is not stored, or corresponding to a special order for new DNA data and/or the DNA chip, (16): the DNA data and/or DNA sample purchasing unit purchases DNA data and/or DNA sample from other provider institutions or the user responding to an order from (12): the storing/managing unit.

In the means for DNA replication of (15): the DNA chip constructing unit, based on the selected data of the user, the provider company performs selection of a specified base sequence area aiming for replicating DNA sample and/or DNA chips which can be provided and, then, performs replication processing. In actual means for replication, a replication method in which the DNA sample is incorporated in a vector system such as a plasmid is possible, but, as described below, the replication is performed by a non-vector system utilizing a mother chip on which DNA library capable of performing an extreme simplification of the replication work and a substantial reduction of a repletion time is immobilized.

Next, in the chip activating unit of (15): the DNA chip constructing unit, a surface of a chip is chemically treated such that the DNA sample subjected to a replication treatment can be immobilized thereon. As for such chips (substrate for use in immobilization), substrates such as, diamond, silicon, and glass are mentioned; particularly, a diamond chip is preferable, because it is excellent in DNA immobilization capacity or stability.

By using a diamond chip product which has been prepared by first rinsing a surface of a diamond chip and, then, chlorinating a surface carbon atom under an ultraviolet irradiation and, thereafter, performing a chlorine substitution by the ultraviolet irradiation under the presence of an ammonia gas to thereby aminate the surface carbon atom based on a chemical modification, a carboxylated diamond chip is prepared by allowing one of carboxyl groups of a divalent carboxylic acid to form an amide bonding with an amino group on the surface of the diamond chip.

Subsequently, by dipping the thus-prepared carboxylated diamond chip in an oligonucleotide solution in a state in which an unmodified carboxyl group thereof is activated, an activated chip which is arranged such that the olegonucleotide corresponding to immobilization of a desired gene/DNA fragment is chemically modified while allowing the divalent carboxylic acid to be a linker and, then, the desired DNA sample such as gene/DNA fragment can be immobilized on a tip of the thus-modified olegonucleotide is prepared.

Then, in the DNA immobilizing unit of (15): the DNA chip constructing unit, the DNA sample subjected to the replication treatment is actually immobilized on the activated chip which has been arranged such that the DNA sample can be immobilized by chemically modifying the olegonucleotide. A reaction in which the DNA sample subjected to the replication treatment is bonded to an immobilized oligonucleotide based on a covalent bonding is performed by utilizing an ordinary kit reagent based on a molecular biology and a relevant enzyme.

On this occasion, the DNA sample such as the gene/DNA fragment for use in constructing the DNA chip to be provided is stored in the DNA sample storing unit of (12): the storing/managing unit in a form of the DNA sample immediately after newly purchased which is not immobilized on a chip or in a form of a mother chip (with the latter form being dominant).

On the other hand, in a case of a request for a special order of new DNA data and/or DNA chip from the user, the mother chip does not exist. Then, the DNA sample which has newly been purchased through (16): the DNA data and/or DNA sample purchasing unit is immobilized on the immobilized olegonucleotide immediately after being purchased in a same manner as described above in an individual form of the gene/DNA fragment, or in a form of a library state by utilizing the ordinary kit reagent based on a molecular biology and the relevant enzyme to thereby being immobilized as a mother chip.

Ordinarily, the mother chip is cDNA library or gDNA library in a state in which DNA molecules of tens of billions to hundreds of billions per chip are immobilized on one face, after being subjected to such pretreatment for immobilization as described above, of a diamond flitter (for example, 3 mm x 3 mm) made of a highly thermally conductive thin film (for example, 0.1 mm to 0.2 mm thick) prepared by a microwave vapor phase synthesis method.

In a case in which (16): the DNA data and/or DNA sample purchasing unit of the apparatus for providing DNA data/chips purchases the DNA sample as a total RNA sample and, then, constructs cDNA library (one chain), or in a case in which the DNA sample is purchased thereby from the beginning as cDNA library (one chain) from the total RNA sample, the mother chip corresponding to each of these libraries is constructed such that an oligo(dT)n chip, having from one to three adenosine or cytidine at a 5' terminal, which has been immobilized on the activated diamond chip on the side of the 5' terminal is dipped in a prepared total RNA solution and, then, a reverse transcriptase is added thereinto. (A portion of the total RNA sample is all the time stored and managed in the DNA sample storing unit of (12): the storing/managing unit.) Replica chips corresponding to 20 or more mother chips can be constructed within a day by an apparatus for automatically constructing replicas which is capable of performing an immobilization method same as that of recycling mRNA to be performed at the time the mother chip is constructed. Changing cDNA library (one chain) into that in two-chain form can relatively easily be performed on the mother chip or the replica chip when needed.

On the other hand, in a case in which (16): the DNA data and/or DNA sample purchasing unit of the apparatus for providing DNA data/chips purchases the DNA sample as a gDNA sample (two chain), after it is treated with an appropriate restrictive enzyme, a chip for immobilizing a two-chain DNA on which olegonucleotide having a same restrictive enzyme portion is immobilized is acted on by ligase to thereby construct the mother chip. (A part of gDNA sample is all the time stored and managed in the DNA sample storing unit of (12): the storing/managing unit.) It is also possible to construct the replica chip by using the thus-constructed mother chip in the apparatus for automatically constructing replicas.

These mother chip and replica chip are stored and managed in the DNA sample storing unit of (12): the storing/managing unit of the apparatus for providing DNA data/chips on the basis of an identification number (mother chip ID number) which is engraved on the back of each of the chips.

By utilizing the mother chip and replica chip of each of the thus-stored and managed cDNA library and gDNA library, an individual gene or DNA fragment contained in each of these libraries is subjected to a PCR (Polymerase Chain Reaction) amplification and, then, the resultant amplified product is immobilized on an activated individual diamond chip in a same manner as described above to thereby construct a daughter chip. The thus-constructed daughter chip is stored in the DNA chip storing unit of (12): the storing/managing unit of the apparatus for providing DNA data/chips on the basis of an identification number (daughter chip ID number) also engraved on the back thereof.

Storing individual DNA data purchased via the communication line (L2) or the DNA data along with the DNA sample purchase and storing the management of information the mother chip, the replica chip and the daughter chip are performed by the DNA data storing unit of (12): the storing/managing unit of the apparatus for providing DNA data/chips.

(17): the DNA chip data analysis unit performs data analysis of the DNA chip on which the DNA sample which has been purchased is immobilized. The data analysis may be all the time performed or performed when the user desires.

### (4) DNA providing process by "Gene Net Bank (GNB)"

Next, a handling method of the apparatus for selecting and purchasing DNA data/chips and the apparatus for providing DNA data/chips of the aforementioned DNA providing system "Gene Net Bank (GNB)" will be described below with reference to the DNA providing system as described in FIG. 2 and flowcharts as described in FIGS. 3 to 5.

### [Main system]

FIG. 3 shows a series of flows of [main system] up to transmission/shipment of DNA data and/or DNA chips.

Firstly, a signal is sent from (3): the selected data inputting unit of the apparatus for selecting and purchasing DNA data/chips to the controlling unit and, then, provided data is opened from (2): the provided data accumulating/selecting unit. Such operation is conducted by a user. When the user does not have an apparatus for selecting/purchasing DNA data/chips at hand, the user goes to a place where the apparatus is installed.

The user selects a desired provided data from (2): the provided data accumulating/selecting unit and, for example, pushes a button-type switch and, then, the controlling unit confirms whether or not the thus-selected DNA data and/or DNA chips (selected data) are within a range of the provided data.

For example, when the user selects DNA data and/or DNA chips related with a given disease or the like, the controlling unit answers back to the user to confirm the desired provided data and, then, recognizes them.

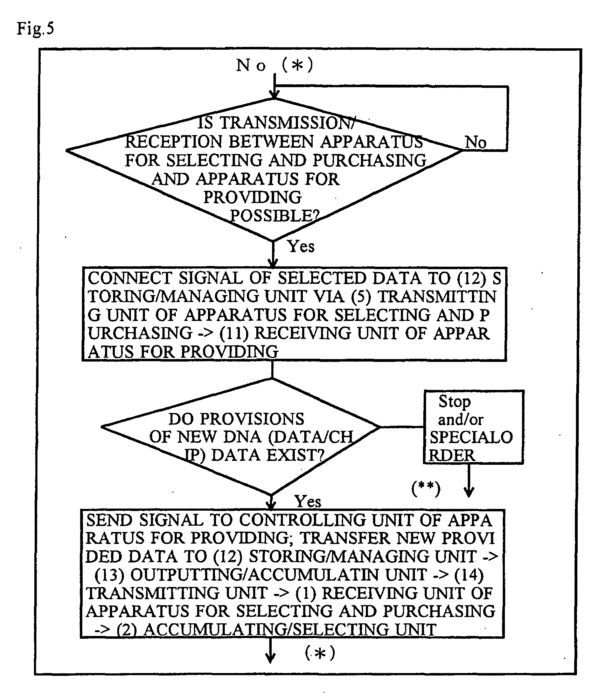

When such selected data are within the range of the provided data (Yes in FIG. 3), procedures proceed to a next step. On the other hand, when the selected data are not within the range of the provided data (No (*) in FIG. 3), the selected data can not be accepted as they are and procedures proceed to a step of [auxiliary system] to be described in FIG. 5.

Next, the desired provided data is read out from (2): the provided data accumulating/selecting unit and the selected data are outputted from (5): the selected data transmitting unit via (4): the selected data outputting/accumulating unit.

Namely, when the user inputs the desired provided data as the selected data in (3): the selected data inputing unit from among the desired provided data from (2): the provided data accumulating/selecting unit, (4): the selected data outputting/accumulating unit enables an output of such data and accumulates thus-enabled data and, thereafter, such data are outputted from (5): the selected data transmitting unit.

Subsequently, when transmission/reception are possible between the apparatus for selecting and purchasing DNA data/chips and the apparatus for providing DNA data/chips (Yes), procedures proceed to a next step.

Next, a signal of the selected data from the apparatus for selecting/purchasing DNA data/chips are sent to the controlling unit via (11): the selected data receiving unit of the apparatus for providing DNA data/chips and, then, the desired DNA chip storing data and/or DNA data from (12): the storing/managing unit is read out and, thereafter, the thus-read data are sent to (1) the provided data receiving unit of the apparatus for selecting/purchasing DNA data/chips via (13): the provided data outputting/accumulating unit, and (15): the provided data transmitting unit.

The term "DNA chip storing data" as used herein means data indicating that the DNA chip is in stock, or data indicating that, although the mother chip or the replica chip corresponding to the DNA chip or the DNA sample exists, the DNA chip is not in stock in the DNA chip storing unit.

When the user desires DNA data, under a condition that transmission/reception is possible between the apparatus for selecting and purchasing DNA data/chips and the apparatus for providing DNA data/chips (Yes), corresponding DNA data read out from the apparatus for providing DNA data/chips are transmitted.

The user can receive the desired DNA data via the receiving unit of the apparatus for selecting and purchasing DNA data/chips.

On the other hand, in a case in which the user desired the DNA chip, when the desired DNA chip is stored (in stock) (Yes), the DNA chip is sent to the user.

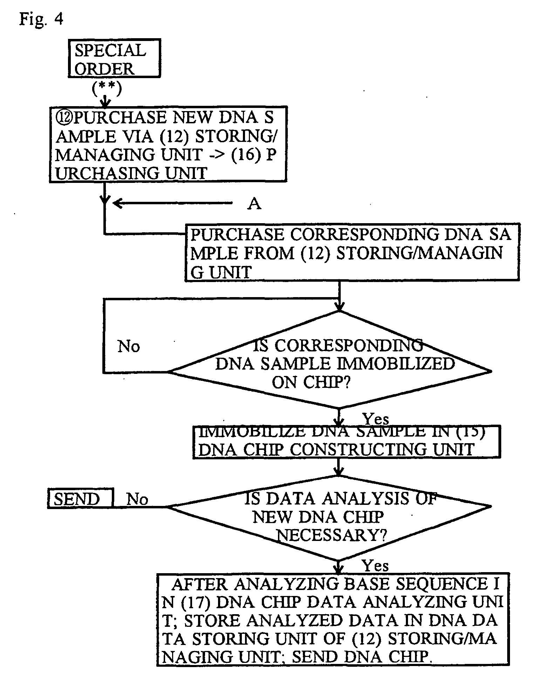

When the desired DNA chip is not stored (out of stock), the procedures proceed to a flow as described in FIG. 4 of [auxiliary system].

Further, when the user desires both the DNA data and the DNA chip, in the process as described above, the DNA chip along with the DNA data is sent from the DNA chip storing unit of (12): the storing/managing unit to the user. At the time of shipment, an invoice is attached thereto to request for payment thereof.

### [Auxiliary system]

### [1] In a case in which, although the mother chip or the replica chip corresponding to the DNA chip selected by the user or the DNA sample exists, the DNA chip is not in stock in the DNA chip storing unit (FIG. 4)

FIG. 4 shows a series of flows of process of DNA chip construction/shipment in a case in which a DNA chip selected by a user is not in stock.

When a desired DNA chip is not stored (out of stock), (12): the storing/managing unit of the apparatus for providing DNA data/chips purchases a material short in stock such as DNA sample. Specifically, purchase is requested to (16): the DNA data and/or DNA sample purchasing unit and, then, not only a DNA sample but also a chip material or a material necessary for constructing a DNA chip is requested to other providing institutions or the user to thereby performing the purchase.

When the DNA sample corresponding to the chip can be immobilized (Yes), the DNA sample is immobilized in (15): the DNA chip constructing unit to thereby constructing a daughter chip. Namely, when (16): the DNA data and/or DNA sample purchasing unit purchases the DNA sample or the like, (12): the storing/managing unit receives instructions and, then, not only sends the DNA sample but also gives instructions for constructing the DNA chip to (15): the DNA chip constructing unit.

When the data analysis of the thus-constructed DNA chip is not necessary (No), the constructed DNA chip is sent to the user. At the time of shipment, an invoice is attached thereto to request for payment of the charge thereof.

On the other hand, when data analysis of the DNA chip is necessary (Yes), an analysis of base sequence is performed in (17): the DNA chip data analysis unit and, then, not only the thus-analyzed data are stored in the DNA data storing unit of (12): the storing/managing unit but also the DNA chip is sent.

### [2] In a case in which new DNA data and/or DNA chips which do not exist in the provided data are requested (FIG. 5)

On the other hand, FIG. 5 shows a treatment flow in a case in which DNA data and/or DNA chips desired by the user do not exist in the provided data.

When DNA data and/or DNA chips desired by the user do not exist in the provided data of (2): the provided data accumulating/selecting unit of the apparatus for selecting and purchasing DNA data/chips (No (*)), under a condition that transmission/reception is possible between the selecting and purchasing apparatus and the providing apparatus (Yes), a signal of the selected data is connected from (5): the selected data transmitting unit of the apparatus for selecting and purchasing DNA data/chips to (11) : the selected data receiving unit of the apparatus for providing DNA data/chips and is transferred to (12) : the storing/managing unit of the apparatus for providing DNA data/chips as new data for requesting a provision.

Further, the signal of the selected data is such that, in regard to the new DNA data and/or DNA chips inputted in (3): the selected data inputting unit by the user in a same manner as in an ordinary manner, (4): the selected data outputting/accumulating unit enables an output of such data and, then, accumulates the thus-enabled data and, thereafter, the selected data transmitting unit outputs such data outward as a signal.

Subsequently, whether or not new DNA data and/or DNA chips corresponding to the selected data exist in the DNA data storing unit for (12): the storing/managing unit, the DNA sample storing unit, and the DNA chip storing unit is inquired.

When the corresponding new DNA data and/or DNA chips are added in (12): the storing/managing unit, the controlling unit of the apparatus for providing DNA data/chips sends the fact back to (2): the provided data accumulating/selecting unit of the apparatus for selecting and purchasing DNA data/chips via (13): the provided data outputting/accumulating unit and (14): the provided data transmitting unit and, then, such data are additionally inserted in the provided data and, at the same time, the DNA data and/or DNA chips desired by the user are received as selected data (back to (*) of FIG. 3, and the aforementioned process is repeated). On the other hand, when the corresponding new provided data do not exist (No), the user gives up purchasing and stops an entire communication (Stop) or request a special order for the new DNA data and/or DNA chips ((**) of FIG. 4).

(12): the storing/managing unit of the apparatus for proving DNA data/chips which receives a special order for new DNA data and/or DNA chips finds new DNA data desired by the user, or new DNA data and/or DNA sample from the side of the provider of DNA data and/or DNA sample through the purchasing unit to purchase it.

Then, new DNA chip is constructed as a mother chip and, thereafter, a daughter chip thereof is constructed and, subsequently, sent to the user along with the DNA data and, at the same time, several spares thereof are stored in the DNA chip storing unit of (12): the storing/managing unit.

### Industrial Applicability

An operation of constructing a DNA chip in a DNA providing system "Gene Net Bank (GNB)" according to the present invention as described in Claim 1 is performed on the basis of data accumulated in (12): the storing/managing unit of the apparatus for providing DNA data/chips, and the user can obtain the DNA data/DNA chips without being restricted by factors such as time, place or environment and, accordingly, can conduct an efficient research or a medical diagnosis.

Further, according to the present invention as described in Claim 1, by only selecting a function of DNA, the user can immediately obtain base sequence data of DNA having the function or the DNA chip and, accordingly, can conduct a research or a medical diagnosis.

Still further, according to the present invention as described in Claim 1, since a DNA data/DNA chip provider company can also quickly receive a user's request, not only a quick service provision is possible, but also an increase of an income derived from an increase in the number of customers can be expected.

Furthermore, by utilizing the system according to the present invention as described in Claim 1, universities, laboratories, companies or the like which have invested a large sum of money, particularly, in a base sequence analysis of DNA or investigation of functions thereof can sufficiently collect such investment.

## Claims

1. A DNA providing system, comprising:
an apparatus for selecting and purchasing DNA data/chips comprising a provided data receiving unit for receiving DNA data and/or DNA chip data (hereinafter referred to as "provided data") provided via a communication line;
a provided data accumulating/selecting unit for accumulating the provided data received by the provided data receiving unit and selecting the provided data desired by a user from among the thus-accumulated provided data;
a selected data inputting unit for inputting the DNA data and/or DNA chip selection data (hereinafter referred to as "selected data") selected by the user from among the provided data of the provided data accumulating/selecting unit;
a selected data outputting/accumulating unit for enabling an output of the selected data selected by the selected data inputting unit, and accumulating the same thus enabled;
a selected data transmitting unit for outputting the selected data from the selected data outputting/accumulating unit outward; and
an indicating unit/controlling unit for indicating and controlling the provided data, the selected data or the like, and
an apparatus for providing DNA data/chips comprising a selected data receiving unit for receiving the selected data from the selected data transmitting unit of the apparatus for selecting and purchasing DNA data/chips;
a storing/managing unit for storing the selected data, the DNA data, DNA samples for the DNA chips, and the DNA chips;
a provided data outputting/accumulating unit for enabling an output of the provided data corresponding to the selected data outward from the storing/managing unit, and accumulating the same thus enabled;
a provided data transmitting unit for outputting the provided data from the provided data outputting/accumulating unit outward;
a DNA chip constructing unit for constructing DNA chips desired by the user when the DNA chips are not stored in a DNA chip storing unit of the storing/managing unit; and
an indicating unit/controlling unit for indicating and controlling the provided data, the selected data or the like.

2. The DNA providing system as set forth in Claim 1, wherein, in the apparatus for selecting and purchasing DNA data/chips, the selected data inputting unit further has a function of inputting new DNA data and/or DNA chips which do not exist in the provided data (hereinafter referred to as "new DNA data and/or DNA chips");
the selected data outputting/accumulating unit has a function of enabling an output of the new DNA data and/or DNA chips and accumulating the same thus enabled; and
the selected data transmitting unit has a function of outputting the new DNA data and/or DNA chips outward.

3. The DNA providing system as set forth in Claim 2, wherein, in the apparatus for providing DNA data/chips, the selected data receiving unit has a function of receiving the new DNA data and/or DNA chips from the selected data transmitting unit of the apparatus for selecting and purchasing DNA data/chips;
when the new DNA data and/or DNA chips which are stored as provided data are in a state of being not transmitted to the apparatus for selecting and purchasing DNA data/chips, the storing/managing unit further has a function of giving instructions for transmitting the same; and
the provided data outputting/accumulating unit further has a function of enabling an output of the new DNA data and/or DNA chips outward from the storing/managing unit as the provided data and accumulating the same thus enabled.

4. The DNA providing system as set forth in any one of Claims 1 to 3, wherein, when DNA samples for constructing the DNA chips desired by the user are not stored, the apparatus for providing DNA data/chips further comprises a DNA data and/or DNA sample purchasing unit for purchasing DNA data and/or DNA samples from other providing institutions or the user.

5. The DNA proving system as set forth in any one of Claims 1 to 4, wherein the apparatus for providing DNA data/chips further comprises a DNA chip data analysis unit for performing a data analysis of DNA chips on which purchased DNA samples are immobilized.

6. The DNA proving system as set forth in any one of Claims 1 to 5, wherein transmission/reception of the selected data between the selected data transmitting unit of the apparatus for selecting and purchasing DNA data/chips and the selected data receiving unit of the apparatus for providing DNA data/chips are conducted in a non-contact manner.

7. The DNA proving system as set forth in any one of Claims 1 to 6, wherein transmission/reception of the provided data between the provided data transmitting unit of the apparatus for providing DNA data/chips and the provided data receiving unit of the apparatus for selecting and purchasing DNA data/chips are conducted in a non-contact manner.

8. The DNA proving system as set forth in any one of Claims 1 to 7, wherein the storing/managing unit of the apparatus for providing DNA data/chips comprises a selected data storing unit, a DNA data storing unit, a DNA sample storing unit, and a DNA chip storing unit.

9. The DNA proving system as set forth in any one of Claims 1 to 8, wherein the DNA chip constructing unit of the apparatus for providing DNA data/chips comprises means for DNA replication, a chip activating unit and a DNA immobilizing unit.
